# EUROPEAN PATENT SPECIFICATION

(11) **EP 1 690 931 B1**
(45) Date of publication and mention of the grant of the patent: **22.01.2014**
(21) Application number: 05012351.2
(22) Date of filing: 08.06.2005
(51) Int. Cl.: C12N 11/04, A23K 1/00, A23K 1/16, A23D 9/00

(54) **Method of delivering a live organism product**
Verfahren zum Auslegen von Zusammensetzung enthaltend lebendende Organismen
Procédé de livraison de produit contentant des organismes vivants

(30) Priority: 10.02.2005 US 55433
(43) Date of publication of application: 16.08.2006
(73) Proprietor: Watson, James B., Pierce, Nebraska 68767 (US)
(72) Inventor: Watson, James B., Pierce, Nebraska 68767 (US)
(74) Representative: Prinz & Partner

(56) References cited:
- EP-A1- 1 364 579
- WO-A-02/00035
- WO-A-92/12234
- WO-A-92/12639
- WO-A-03/053397
- WO-A1-89/07446
- WO-A1-93/17094
- WO-A1-2005/040373
- CH-A- 457 332
- GB-A- 1 151 669
- US-A- 4 161 397
- US-A- 4 518 696
- US-A- 4 647 536
- US-A- 5 624 684
- US-A- 5 725 853
- US-A1- 2002 119 237
- DATABASE WPI Week 198620, Derwent Publications Ltd., London, GB; AN 1986-128930 & JP 61 067488 A (HITACHI PLANT ENG & CONSTR CO) 07 April 1986

## Description

This invention, which is defined by the method of the claims, relates to a dormant organism product and more particularly to a live dormant organism product wherein live organisms, that are in a dormant state, are suspended in a liquid fluid carrier or medium that can be easily pumped and will stay in suspension for the life of the product. More particularly, the carrier, while being in a liquid state, is free of moisture to prevent the organisms from becoming activated until the product is applied to feeds, forages, or directly onto animals or the like. Hereinafter, feeds, forages, animals or the like will be referred to as "the target host".

Live bacteria formulations are frequently applied to forage products, feed, etc., to enhance fermentation and/or digestion. The prior art products fall generally into two categories which are either a dry soluble powder form or a dry granular non-soluble form. The dry granular products are directly applied to its target host through a metering device such as a Gandy box, usually at a rate of 110 to 450 g (4 to 16 ounces) per ton of forage treated. The dry soluble products must first be hydrated with water, then applied to its target host within a 48-hour time period post-hydration with application made through a liquid pump system at the rate of 37 grams to 3.8 l (1 gallon) per ton of forage.

The dry granular products of the prior art, even if packaged in plastic pails, will sometimes come into direct contact with ambient air during manufacturing and processing or upon simply opening the product lid with the ambient air containing moisture or humidity. The moisture or humidity will activate the dormant bacteria upon contact which in turn causes the bacteria to inadvertently live and die before it can be applied to the forage. The value of the product will then be a total loss. The limestone carriers of the prior art products will carry minute traces of moisture, and such moisture severely reduces the product's ability to sustain an adequate shelf life. If the prior art product is a soluble powder product, it will also need to be hydrated before application. The hydration immediately activates all of the dormant bacteria and the producer has to apply the entire mixture within a 48-hour time frame or before since the bacteria will naturally deplete its food source and die, creating a total loss of the product value.

Further, the prior art products on the market today must be either removed from their original shipping container or physically poured into a dry applicator prior to application, or they must be contaminated with water, reducing their shelf life to less than 48 hours, post-contamination. Additionally, the prior art dry granular products have a tendency of sorting during shipment. This shakes any fines to the bottom of the shipping container leaving the either larger or lighter material to work its way to the top. Since the bacteria attach themselves to these particles, they may also migrate with these particles. Other dry soluble powder products may settle to the bottom of the applicator tank upon hydration. This will depend greatly on the product's rate of dilution. As application rates decrease to meet the producer's ongoing needs of hauling less product to the field, the manufacturers must in turn make their products more condensed to meet these needs. Such limits the solubility of the product.

The dry granular products of the prior art require a significant percentage of the product to be applied to the material which results in uniform coverage becoming difficult. Dry soluble powders mixed with water carriers have attempted to achieve low inclusion rates for some time. However, such products result in droplets which are very large in their molecular size and weight resulting in that there are actually few droplets being delivered per treatment, an example being one ton of forage treated with only ten large droplets so uniform coverage is poor.

WO 93/17094 discloses the use of microspheres as a carrier for microorganisms, where the mcroorganisms are preferably a bacteria. The microspheres are created in a method of rotary disk production, where the microspheres are ejected from the disk at high rotary speed. A mixture of a fatty acid melt and the organisms is applied to the rotary disk to produce the coated spheres, which at room temperature return to the solid state to provide an effective matrix. The fatty acid is a stearic acid.

WO 89/07446 discloses a method for desiccation of biological organisms in oil suspension. Nematodes which are parasitic to one or more species of insects are suspended in a suitable oil, which may be a vegetable, mineral or synthetic oil. The biological material can also be a viable virus or a bacteria. The desiccating agent is a water absorbent matrix material.

FR 457 332 discloses a dormant microorganism in an anhydrous media, the carrier medium being a vegetable oil, such as olive oil, or a fatty acid such as margarine. The fatty acid can also be a glyceride.

GB 1,151,669 discloses a method of treatment of freeze-dried bacteria cultures. The cultures are embedded in a liquid medium comprised of a saturated fatty acid, in particular glycerides which are liquid at room temperature.

WO 92/12639 discloses a method of supplying microorganisms to animals in freeze dried form. It was found that organisms were suspended in sunflower oil, there was no loss of viability.

It is therefore a principal object of the invention to provide an improved live dormant organism product which requires ambient moisture to begin proliferation.

Yet another object of the invention is to provide a live dormant organism product wherein live organisms, that are in a dormant state, are suspended in a clear liquid fluid carrier or medium that can be easily pumped and will stay in suspension for the life of the product.

Still another object of the invention is to provide a live dormant organism product which is free of moisture to prevent the organism from becoming activated until the product is applied to the target host.

Still another object of the invention is to provide a live dormant organism product which may be applied to a target host at an extremely small rate.

Yet another object of the invention is to provide a live dormant organism product which may be applied to forage or feed at a preferred micro-treatment rate.

According to the present invention, a method of forming and applying a stabilized admixture of dormant viable organisms is provided as defined in claim 1.

The product obtained by the method of this invention is applied to the target host at a micro-treatment rate of 2 to 28 grams per ton of target host being treated. The live bacteria, by being suspended in the moisture-free carrier, will remain dormant until coming into contact with the target host at which time the moisture in the target host will cause the bacteria to be activated. The product is packaged in controlled shipping vessels such as polyurethane bags that reduce the risk of moisture contamination of any kind. The product is sold and shipped directly to the producer who will then take the collapsible bag of product and hook it directly to an applicator which applies the product to the target host. The target host may be forage, feed or the like. In some cases, the product may be sprayed directly onto animals such as chicks who will ingest the product while grooming themselves. Although the product is ideally an improve live bacterial product, the product may be freeze dried live dormant organisms that require ambient moisture to begin proliferation including, but not limited to, modified live bacterial and viral vaccines such as Parvo, Bovine Rhinotracheitis, Leptospira, BVD, IBR and P13 or modified and non-modified pathogens.

### DETAILED DESCRIPTION OF EMBODIMENTS

In the following, the method of this invention is described for a live dormant bacteria product. However, it should be understood that the product could be: (1) a live dormant organism requiring ambient moisture to begin proliferation; (2) modified or live bacterial and viral vaccines such as Parvo, Bovine Rhinotracheitis, Leptospira, BVD, IBR and P13; or (3) modified and non-modified pathogens.

In this method, conventional live viable harmless bacteria is mixed with a substantially moisture-free liquid carrier or medium. In general, the live bacteria will be any lactic acid producing bacteria that is permissible for use in animal related products. The United States Food and Drug Administration (FDA) and The American Association of Feed Control Officials (AAFCO) have published a list of the microorganism species which are "generally recognized as safe" (GRASS) for use in direct-fed microbial products. Table I hereinbelow lists presently approved bacteria for use with animal related products.

**TABLE I**

| **36.14 -- DFM Microorganisms under the "GRASS' Status** | |
|---|---|
| Aspergillus niger | Lactobacillus curvatus |
| Aspergillus oryzae | Lactobacillus delbruekii |
| Bacillus coagulans | Lactobacillus fermentum |
| Bacillus lentus | Lactobacillus helveticus |
| Bacillus licheniformis | Lactobacillus lactis |
| Bacillus pumilus | Lactobacillus plantarum |
| Bacillus subtilis | Lactobacillus euterii |
| Bacteroides amylophilus | Leuconostoc mesenteroides |
| Bacteroides capillosus | Pediococcus acidilacticii |
| Bacteroides ruminocola | Pediococcus cervisiae |
| Bacteroides suis | Pediococcus pentosaceus |
| Bifidobacterium adolescentis | Propionibacterium freudenreichii |
| Bifidobacterium animalis | Propionibacterium shermanii |
| Bifidobacterium bifidum | Saccharomyces cerevisiae |
| Bifidobacterium infantis | Entercococcus cremoris |
| Bifidobacterium longum | Entercococcus diacetylactis |
| Bifidobacterium thermophilum | Entercococcus faecium |
| Lactobacillus acidolphilus | Entercococcus intermedius |
| Lactobacillus brevis | |
| Lactobacillus buchneri | Entercococcus lactis |
| Lactobacillus bulgaricus | Entercococcus thermophilius |
| Lactobacillus casei | Yeast (as defined elsewhere) |
| Lactobacillus cellobiosus | |

Preferably, the moisture-free liquid carrier is comprised of a product marketed under the trademark "Synergel®" manufactured by Penreco of 138 Petrolia Street, Karns City, Pennsylvania 16041-9799. The product is generally referred to as a gelled mineral oil, reference number 1004-100, having a Brookfield viscosity of 1242 cPs. The carrier product is clear, odorless and is insoluble in water. The carrier also includes polymers. Other suspension agents such as Cobisal (polymer fiber) may also be used from time to time to aid or reduce the possibility of the settling of the bacteria therein. While the moisture-free oil described above is preferred, a processed oil obtained from either animal, vegetable or petroleum may be used. The product is designed to only activate the bacteria at the physical point of contact with the target host. Upon contact, the target host ambient moisture content will activate the dormant bacteria found within the liquid microbial medium or carrier. The liquid microbial medium or carrier has undergone a special production process developed by Penreco. The process consists of first passing the mineral oil through an advanced filtration system which specifically targets and absorbs any ambient moisture. Secondly, the mineral oil is heated and polymers are added to increase the oil viscosity and suspension qualities. Thirdly, the oil is packaged into controlled shipping vessels that reduce the risk of moisture contamination of any kind.

Upon receipt of the packaged carrier, applicant then preferably adds a moisture scavenger product such as a hydrophilic molecular sieve adsorbent as an insurance program. The adsorbent may comprise a natural or synthetic zeolite consisting of crystalline metal aluminosilicate, alkali metal aluminosilicate or sodium aluminosilicate. Finally, whatever bacteria are needed for a particular purpose will then be added. The final blend is then packaged in moisture and ultraviolet retardant containers such as collapsible polyurethane bags, very similar to I.V. bags. The product is then sold and shipped directly to the retailer and/or end user. The end user will then take the collapsible bag of liquid microbial blend and hook it directly to an applicator so that extremely small droplets are created which are sprayed upon the target host

The method includes the ability to inoculate other products with dormant live bacteria, by means of a light spraying application. Whereas the bacteria is protected with the oil/polymer blend allowing the host bacteria to survive longer in a non-favorable environment, the coating of the bacteria cell walls with the oil/polymer covering provides a physical chemical moisture barrier. The above provides the ability to permit the live bacteria to be introduced onto a pelleted feed without immediately inadvertently activating the oil/polymer/host bacteria through ambient moisture contamination from the feed itself.

According to the method, the product is applied to its target host at a micro-treatment rate of 2 to 28 grams per one ton of forage or the material treated (with no additional water or carriers needed). Such micro-treatment is substantially less than prior art products that have treatment rates that range from 37.88 grams to 3.78 kg (8.3453 pounds) per one ton of forage treated.

The packaging limits product exposure to possible contamination from outside sources, such as moisture-laden air or fluid water of any kind. The product is designed to only activate at the physical point of contact with the target host. Upon contact with the target host, the change in pH and moisture activates the dormant bacteria.

Thus it can be seen that the invention accomplishes at least all of its stated objectives.

## Claims

1. The method of forming and applying a stabilized admixture of dormant viable organisms to animal feed or forage as a target host, said method comprising:
preparing an admixture by intermixing a substantially moisture-free liquid carrier and the dormant organisms, wherein the liquid carrier comprises mineral oil and polymers and wherein the polymers are a suspension agent for the dormant organisms, and
spraying the admixture upon the target host at a rate of 2 to 28 grams per ton of the target host.

2. The method of claim 1, wherein the admixture is sprayed onto the target host having a moisture content and pH value to cause the activation of the organism.

3. The method of claim 1 or2, wherein said admixture includes a hydrophilic molecular sieve adsorbent.

4. The method of claim 3, wherein said adsorbent comprises a natural or synthetic zeolite.

5. The method of claim 4, wherein said zeolite comprises crystalline metal aluminosilicate.

6. The method of claim 4, wherein said zeolite comprises alkali metal aluminosilicate.

7. The method of claim 4, wherein said zeolite comprises sodium aluminosilicate.

8. The method of any one of the claims 1 to 7, wherein the organism comprises a live bacterial vaccine.

9. The method of any one of the claims 1 to 7, wherein the organism comprises a live viral vaccine.

10. The method of any one to the claims 1 to 7, wherein the organism comprises a modified or non-modified pathogen.

## Patentansprüche

1. Verfahren zur Bildung und Anwendung einer stabilisierten Beimischung ruhender, lebensfähiger Organismen zu Tierfutter oder Viehfutter als Zielwirt, wobei das Verfahren umfasst:
Herstellen einer Beimischung durch Vermischen eines im Wesentlichen feuchtigkeitsfreien, flüssigen Trägers und der ruhenden Organismen, wobei der flüssige Träger Mineralöl und Polymere umfasst und wobei die Polymere ein Suspensionsmittel für die ruhenden Organismen sind, und
Besprühen des Zielwirts mit der Beimischung mit einer Rate von 2 bis 28 Gramm pro Tonne des Zielwirts.

2. Verfahren nach Anspruch 1, bei dem die Beimischung auf den Zielwirt gesprüht wird, der einen Feuchtigkeitsgehalt und einen pH-Wert derart hat, dass die Aktivierung des Organismus bewirkt wird.

3. Verfahren nach Anspruch 1 oder 2, bei dem die Beimischung ein hydrophiles Molekularsiebadsorbens aufweist.

4. Verfahren nach Anspruch 3, bei dem das Adsorbens einen natürlichen oder synthetischen Zeolithen umfasst.

5. Verfahren nach Anspruch 4, bei dem der Zeolith kristallines Metallaluminosilicat umfasst.

6. Verfahren nach Anspruch 4, bei dem der Zeolith Alkalimetallaluminosilicat umfasst.

7. Verfahren nach Anspruch 4, bei dem der Zeolith Natriumaluminiumsilicat umfasst.

8. Verfahren nach einem der Ansprüche 1 bis 7, bei dem der Organismus einen lebenden, bakteriellen Impfstoff umfasst.

9. Verfahren nach einem der Ansprüche 1 bis 7, bei dem der Organismus einen lebenden Virusimpfstoff umfasst.

10. Verfahren nach einem der Ansprüche 1 bis 7, bei dem der Organismus ein modifiziertes oder nicht modifiziertes Pathogen umfasst.

## Revendications

1. Procédé de réalisation et d'application d'un adjuvant stabilisé d'organismes viables dormants à de l'alimentation animale ou du fourrage en tant qu'hôte cible, le procédé comprenant :
la préparation d'un adjuvant en mélangeant un support liquide sensiblement exempt d'humidité et les organismes dormants, le support liquide comportant de l'huile minérale et des polymères, et les polymères étant un agent de suspension pour les organismes dormants, et
la pulvérisation de l'adjuvant sur l'hôte cible à un taux de 2 à 28 grammes par tonne de l'hôte cible.

2. Procédé selon la revendication 1, dans lequel l'adjuvant est pulvérisé sur l'hôte cible présentant une teneur en humidité et une valeur pH de manière à provoquer l'activation de l'organisme.

3. Procédé selon la revendication 1 ou 2, dans lequel l'adjuvant présente un adsorbant à tamis moléculaire hydrophile.

4. Procédé ,selon la revendication 3, dans lequel l'adsorbant comporte une zéolithe naturelle ou synthétique.

5. Procédé selon la revendication 4, dans lequel la zéolithe comprend de l'aluminosilicate de métal cristallin.

6. Procédé selon la revendication 4, dans lequel la zéolithe comprend de l'aluminosilicate de métal alcalin.

7. Procédé selon la revendication 4, dans lequel la zéolithe comprend de l'aluminosilicate de sodium.

8. Procédé selon l'une des revendications 1 à 7, dans lequel l'organisme comprend un vaccin bactérien vivant.

9. Procédé selon l'une des revendications 1 à 7, dans lequel l'organisme comprend un vaccin viral vivant.

10. Procédé selon l'une des revendications 1 à 7, dans lequel l'organisme comprend un pathogène modifié ou non-modifié.
